# EUROPEAN PATENT APPLICATION

(11) **EP 4 155 382 A1**
(43) Date of publication of application: **29.03.2023**
(21) Application number: 21306334.0
(22) Date of filing: 27.09.2021
(51) Int. Cl.: C12N 1/20, A61K 35/74, A61K 35/741

(54) **BIOLOGICAL CONTROL OF VIBRIOSIS IN AQUACULTURE**

(71) Applicant: Centre national de la recherche scientifique, 75016 Paris (FR); Université de Bretagne Occidentale, 29200 Brest (FR)
(72) Inventor: PICHEREAU, Vianney, 29200 Brest (FR); PAILLARD, Christine, 29840 Porspoder (FR); DELAVAT, François, 44130 Notre Dame Des Landes (FR); RAHMANI, Alexandra, 29810 Breles (FR); LE CHEVALIER, Patrick, 29950 Clohars-Fouesnant (FR)
(74) Representative: Cabinet Becker et Associés

(57) **Abstract**

The present invention relates to the biological control of vibriosis in aquatic animals such as molluscs, that are notably produced in aquaculture for human consumption. The present invention also relates to a particular bacterial strain, and compositions thereof, that are useful for controlling vibriosis in aquatic animals.

## Description

### INTRODUCTION

The present invention relates to the biological control of vibriosis in aquatic animals such as molluscs, that are notably produced in aquaculture for human consumption. The present invention also relates to a particular bacterial strain, and compositions thereof, that are useful for controlling vibriosis in aquatic animals.

Almost 16 million metric tons of molluscs are cultured each year on a global scale. In Europe, mollusc aquaculture in hatcheries is a traditional, well-established industry, essential to the socio-economic development of most coastal regions. The worldwide decline of natural beds has further reinforced the importance of hatchery production to satisfy an ever-increasing demand of consumers. Unfortunately, this type of aquaculture is frequently affected by serious diseases outbreaks, mainly related to bacterial infections. These outbreaks can lead to a loss of complete batches of production and thus represent a major threat to the economic viability of this industry.

Classical approaches employed to control these diseases typically rely on the elimination of microbiota from the culture water via various methods ranging from water treatment (e.g., filtration, disinfection with chlorine, ozonization, UV-radiation) to chemotherapy (use of antibiotics such as chloramphenicol, combistrep, etc.). These methods however have a rather limited effectiveness, a high economic cost, and can be complex to implement. The complete elimination of microbiota was actually shown to favor the colonization of the aquaculture system by opportunistic bacteria with fast growth rates, such as members of the *Vibrio* genus. That is because (1) the elimination of microbiota reduces the natural competition among the bacterial population present in the water leaving an ecological niche empty, and (2) the regular provision of organic matter to feed the molluscs can favor the entry of bacteria into the culture system.

There is thus a need in the art to control the proliferation of pathogens in aquaculture of aquatic animals such as molluscs, in order to efficiently prevent, alleviate, or cure the disorders associated therewith, in a cost-effective manner.

Particular pathogens of interest to be controlled are pathogenic bacteria of the *Vibrio* genus which are associated with the disease *"vibriosis"* that is responsible for mass mortalities in cultured aquatic animals. Among vibrioses that gravely affect molluscs hatcheries, the Brown Ring Disease (BRD) has plagued the French aquaculture since 1986, especially the production of clams such as *Venerupis philippinarum,* a species native of Japan and introduced in France in the 1970s. BRD develops following infection by the bacterium *Vibrio tapetis* and is characterized by the formation of a brown organic conchiolin deposit between the edge of the mollusc shell and the pallial line (Paillard et al., 1989, Comptes rendus l'Académie des Sci., 3: 309, 235-241; Paillard et al., 1994, Annual review of Fish diseases, 4: 219-240). A number of pathogenic *Vibrio tapetis* strains have been isolated not only from diseased clams since the 1990s throughout Europe (Paillard and Maes, 1990, Comptes rendus l'Academie des Sci.310, 15-20; Paillard et al., Annual review of Fish diseases, 4: 219-240, Paillard et al. 2008, Dis. Aquat. Organ., 81, 153-161; Novoa et al., 1998, J. Invertebr. Pathol. 71, 34-41; Allam et al., 2000, Dis. Aquat. Organ., 41:105-113), but also from other marine animals, including fishes (Jensen et al., 2003, Dis. Aquat. Organ., 53 :25-31; Reid et al., 2003, Aquaculture, 221 :65-74; López et al., 2011, Res. Vet. Sc., 90: 189-195; Declercq et al., 2015, Dis. Aquat. Organ. 115, 81-86; Levican et al., 2016, Int. J. Syst. Evol. Microbiol., 67 : 716-723), and crustaceans (Pass et al., 1987, Aquaculture, 65: 149-169 ; Friedman et al., 1991, Aquaculture, 9: 1-15 ; Le Roux et al., 2002, Aquat. Living Resour., 15: 251-258).

The present invention addresses the above discussed need in the art.

In recent years, non-conventional approaches have been proposed, but have yet to be integrated into hatcheries: these include, *inter alia*, the use of probiotics, quorum quenching or phage therapy, to name a few.

Probiotics in the form of live microbial additive are particularly adapted to control the proliferation of pathogens in aquaculture in that, when used as a pre-treatment prior to experimental infection with vibrio, they greatly enhance the survival ratio of molluscs. The beneficial effect of these probiotics may be reached by different mechanisms, such as nutritional complement, improvement of the use of feed, enhancement of the immune response, antibacterial activity, competence for substances, energy or adhesion sites, stimulation of biological processes, and/or improvement of the quality of the water. A desirable probiotic strain suitable for aquaculture of molluscs would typically be a live microorganism (i) originating from the environment where it will be used, herein a marine bacterium, preferably autochthonous from hatchery; that (ii) has a beneficial effect on the target organism, in particular by controlling pathogens; and which (iii) lacks pathogenicity and toxicity, not only for the target organism, but also for other live organisms that are present in the environment. Non-limiting examples of probiotics useful in aquaculture include live microorganisms of a wide range of different bacterial taxa such as *Bacillus sp., Neptunomonas sp., Pseudomonas sp., Alteromonas sp., Pseudoaltermonas sp.*

Without being bound by theory, the Inventors have herein identified a live microorganism that exhibits all the above desirable hallmarks of probiotics, and which can be used to efficiently control a particular vibriosis affecting molluscs hatcheries, namely the Brown Ring Disease. This beneficial microorganism, known as *Vibrio tapetis* GDE, was mentioned for the first time by Dias et al. in 2018 (Frontiers in Microbiology; 9 (227): 1- 14), but was not fully characterized at the time, such that it could not be reproduced by the skilled practitioner without undue burden. The *V*. *tapetis* GDE strain has since been herein deposited at the CNCM on the 6^{th} January 2021 under the accession number reference CNCM 1-5635. By contrast to conventional probiotics, this particular bacterial strain belongs to the same genus as the pathogenic bacteria to be controlled, namely the *Vibrio* species.

Because the strain *Vibrio tapetis* CECT4600 has been identified as the etiological bacterial agent of Brown Ring Disease (BRD), affecting particularly clams, the Inventors have herein investigated whether the strain *V*. *tapetis* GDE can have a protective effect against BRD. To do so, they conducted *in vitro* and *in vivo* virulence assays on Manila clams, with the *V*. *tapetis* CECT4600 and GDE strains, alone and in combination. This study shows that the GDE strain can effectivity counteract the cytotoxic effects induced by the CECT4600 strain, and reduce BRD prevalence as well as the BRD extent index.

Thanks to these unexpected properties, the avirulent *V*. *tapetis* GDE strain can be envisioned as a new agent to control vibriosis affecting aquatic animals, that are notably bred in aquaculture for human consumption. To this end, this GDE strain can be provided alone or in a composition, to said animals.

### SUMMARY OF THE INVENTION

In a first aspect, the present invention relates to an isolated *Vibrio tapetis* strain deposited on the 6^{th} January 2021 at the CNCM under the accession number CNCM 1-5635, or a composition thereof, for use in controlling vibriosis in aquatic animals in need thereof.

In a preferred embodiment, the aquatic animals are molluscs, fishes or crustaceans, preferably molluscs.

In a preferred embodiment, the molluscs are clams, preferably *Venerupis sp.* clams, more preferably *Venerupis philippinarum.*

In a preferred embodiment, the aquatic animals are, or intended to be, in aquaculture.

In a preferred embodiment, the vibriosis is mediated by a virulent *Vibrio tapetis* strain, preferably by the virulent *Vibrio tapetis* CECT4600 strain.

In a preferred embodiment, the vibriosis is Brown Ring Disease (BRD).

In a preferred embodiment, the isolated *Vibrio tapetis* strain deposited on the 6^{th} January 2021 at the CNCM under the accession number CNCM 1-5635 is in a live form.

In a preferred embodiment, the isolated *Vibrio tapetis* strain deposited on the 6^{th} January 2021 at the CNCM under the accession number CNCM 1-5635 is used at the same or higher cellular density than the one of the virulent *Vibrio tapetis* strain that induces the vibriosis in the aquatic animals.

In another aspect, the present invention relates to an isolated *Vibrio tapetis* strain deposited on the 6^{th} January 2021 at the CNCM under the accession number CNCM 1-5635, or a composition thereof, for use in promoting the health of aquatic animals in need thereof.

In another aspect, the present invention relates to the use of an isolated *Vibrio tapetis* strain deposited on the 6^{th} January 2021 at the CNCM under the accession number CNCM I-5635, or of a composition thereof, for controlling virulent *Vibrio tapetis* strains, preferably the virulent *Vibrio tapetis* CECT4600 strain, in an aquatic environment, such as in aquaculture.

In another aspect, the invention relates to the use of an isolated *Vibrio tapetis* strain deposited on the 6^{th} January 2021 at the CNCM under the accession number CNCM 1-5635, or of a composition thereof, in the culturing of aquatic animals, such as in aquaculture.

In another aspect, the invention relates to an isolated *Vibrio tapetis* strain deposited on the 6^{th} January 2021 at the CNCM under the accession number CNCM 1-5635.

In another aspect, the invention relates to a composition comprising at least an isolated *Vibrio tapetis* strain deposited on the 6^{th} January 2021 at the CNCM under the accession number CNCM 1-5635 as an active agent.

In a preferred embodiment, the composition is an additive intended for culturing aquatic animals, such as a feed additive.

In a preferred embodiment, the composition is a probiotic composition.

### LEGENDS TO THE FIGURES

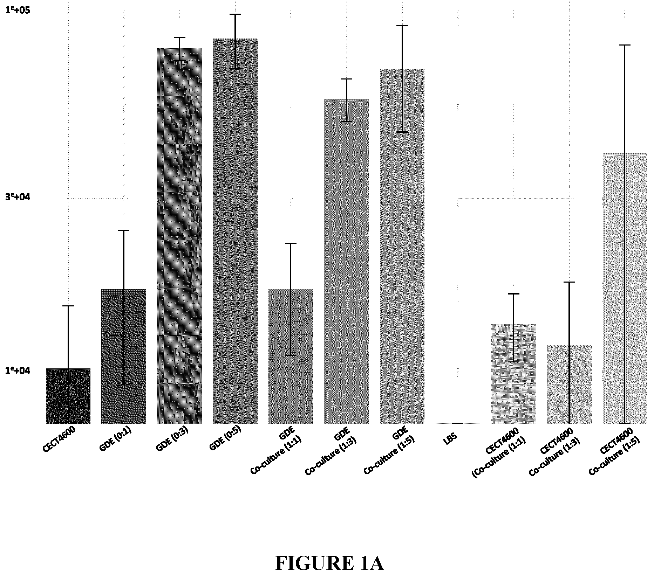

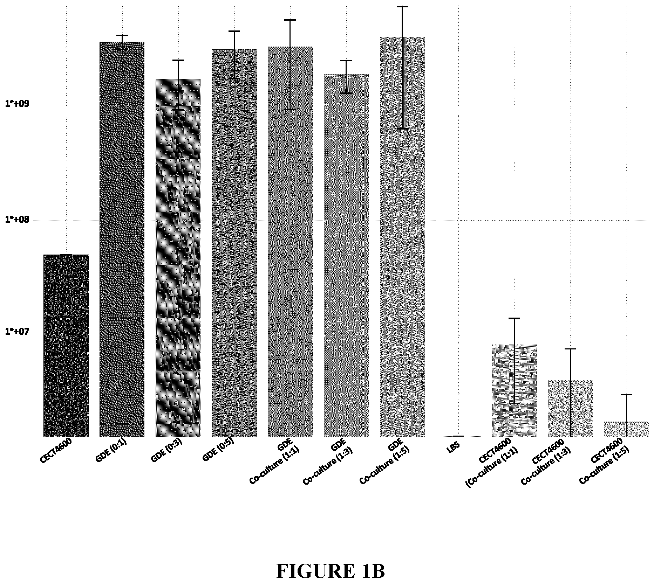

**Figure 1****. *In vitro* competition assay. (A)** The graph indicates the concentration of the *V*. *tapetis* strains at the beginning of the co-cultures The term "*co-culture*" refers to a combination of the CECT4600 and GDE *V. tapetis* strains, at the following initial ratios: 1:1; 1:3; 1:5 A ratio 0:1, 0:3 or 0:5 means that only the GDE strain was cultured at different concentrations. **(B)** The graph indicates the concentration of the *V*. *tapetis* strains after 24h of bacterial growth (TF). The term "*co-culture*" refers to a combination of the CECT4600 and GDE *V*. *tapetis* strains, at the same ratios as in **(A).** In **(A)** and **(B),** LBS is the culture medium without bacteria, used as a control.

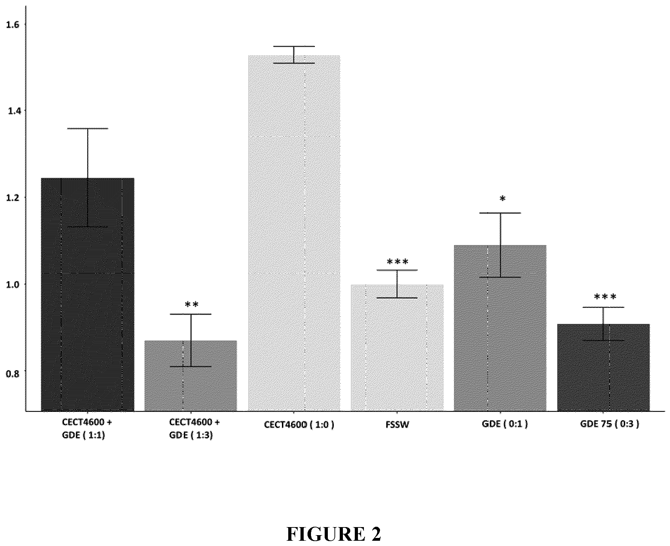

**Figure 2****. *In vitro* virulence assay on Manila claims.** The graph indicates the ratio of non-adherent hemocytes after exposure to FSSW (negative control) alone, to the CECT4600 *V*. *tapetis* strain (positive control) alone, to the GDE *V*. *tapetis* strain alone, and to a combination of the CECT4600 and GDE *V*. *tapetis* strains (co-exposure ratios: 1:1; 1:3). ^{∗}: pvalue <0.05; ^{∗∗}: pvalue <10⁻²; ^{∗∗∗}: pvalue<10⁻³.

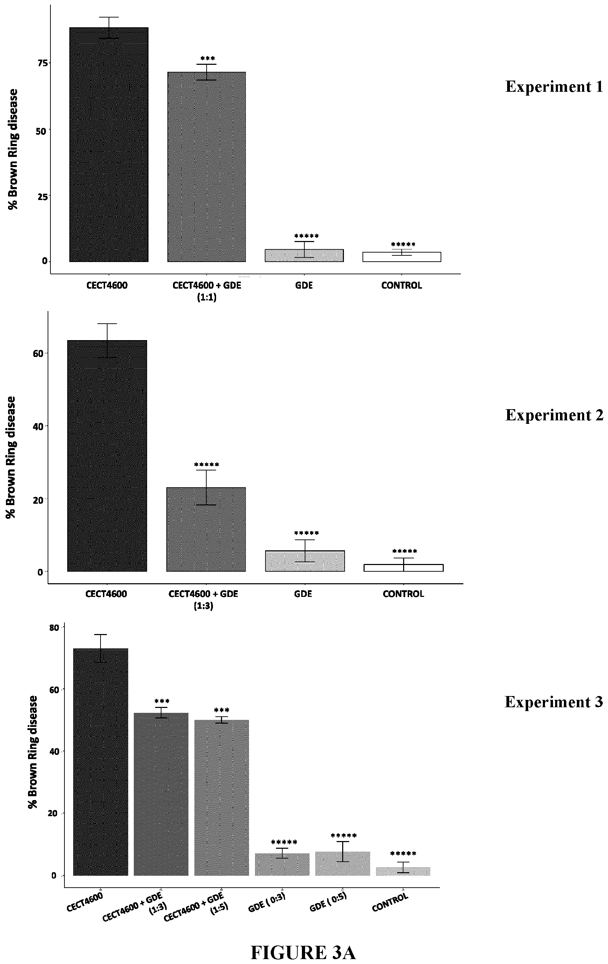

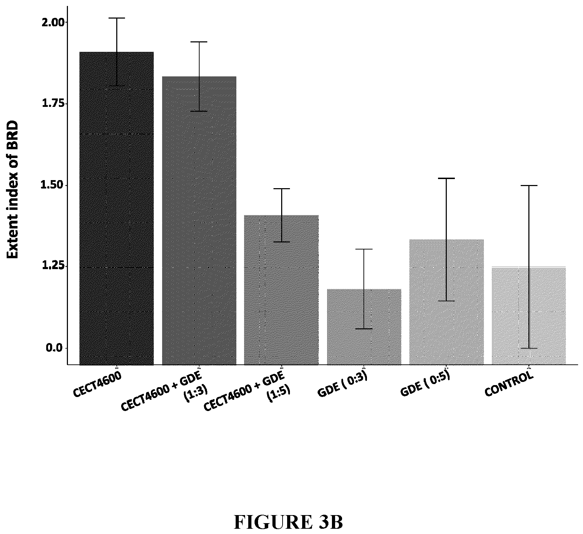

**Figure 3****. *In vivo* virulence assay on Manila claims.** The graphs indicate **(A)** Brown Ring Disease prevalence in 3 independent experiments, and **(B)** the extent index of Brown Ring Disease prevalence, after exposure of Manila clams to FSSW (negative control) alone, to the CECT4600 *V*. *tapetis* strain (positive control) alone, to the GDE *V*. *tapetis* strain alone, and to a combination of the CECT4600 and GDE *V*. *tapetis* strains (co-exposure ratios 1:1, 1:3, 1:5). n=150 for each condition tested. ^{∗∗∗}: pvalue<10⁻³; ^{∗∗∗∗∗}: pvalue <10⁻⁵.

### DETAILED DESCRIPTION OF THE INVENTION

The present invention may be understood more readily by reference to the following detailed description, included preferred embodiments of the invention, and examples included herein.

Unless otherwise defined herein, scientific and technical terms used in connection with the present invention shall have the meanings that are commonly understood by those of ordinary skill in the art. Further, unless otherwise required by context, nomenclatures used herein, and techniques of bacteria preparation are those well-known and commonly used in the art. Nevertheless, whenever necessary, definitions are provided with respect to the use of different terms throughout the current specification.

The Inventors have herein discovered that the *Vibrio tapetis* strain deposited on the 6^{th} of January 2021 at the CNCM under the accession number CNCM 1-5635 is capable of counteracting the clinical signs of vibriosis, both in *in vitro* and *in vivo* models of the disease, notably in clams.

The present invention thus relates to the control of vibriosis in aquatic animals in need thereof, such as molluscs, notably those that are produced in aquaculture for the purpose of human consumption. The present invention further relates to the promotion of health of said aquatic animals, to the control of virulent *Vibrio tapetis* strains in aquaculture in particular of their growth, and to the culturing of aquatic animals. The present invention also relates to the particular bacterial strain deposited on the 6^{th} January 2021 at the CNCM under the accession number CNCM 1-5635, and composition thereof.

Accordingly, in a first aspect, the invention relates to an isolated *Vibrio tapetis* strain deposited on 6^{th} of January 2021 at the CNCM under the accession number CNCM 1-5635, or a composition thereof, for use in controlling vibriosis in aquatic animals in need thereof.

In particular, the present invention is directed to the use of an isolated *Vibrio tapetis* strain deposited on the 6^{th} January 2021 at the CNCM under the accession number CNCM 1-5635, or of a composition thereof, for the preparation of an active agent, such as a medicament, for the control of vibriosis in aquatic animals in need thereof.

It is also described herein a method for controlling vibriosis in aquatic animals in need thereof, said method comprising the step of adding an effective amount of an isolated *Vibrio tapetis* strain deposited on the 6^{th} January 2021 at the CNCM under the accession number CNCM 1-5635, or of a composition thereof, to an environment comprising or intended to comprise aquatic animals (i.e. an aquatic environment).

To do so, one can, for example, bath aquatic animals with the isolated *Vibrio tapetis* strain deposited on the 6^{th} January 2021 at the CNCM under the accession number CNCM 1-5635, or a composition thereof. Alternatively, one may, for example, add the isolated *Vibrio tapetis* strain deposited on the 6^{th} January 2021 at the CNCM under the accession number CNCM I-5635, or a composition thereof, to the environment, prior to the introduction of the aquatic animals into said environment.

In the context of the present invention, the *V*. *tapetis* strain deposited on the 6^{th} January 2021 at the CNCM under the accession number CNCM 1-5635 is said to be isolated.

By *"isolated"*, it is meant that the bacterial strain described herein is provided in a form that is removed from its native environment in which it is naturally present, that is, the bacterial strain is removed (or essentially removed) from one or more or all of the naturally occurring constituents with which it may be associated in nature. In other words, the *V*. *tapetis* strain deposited on the 6^{th} January 2021 at the CNCM under the accession number CNCM 1-5635 is herein provided in a biologically form that is pure, or at least substantially pure.

Besides, for the purpose of the present invention, it should be understood that the *V*. *tapetis* strain deposited on the 6^{th} January 2021 at the CNCM under the accession number CNCM 1-5635 is preferably provided or used in a live form.

By "*live form*"*,* it is meant herein that the bacterial strain comprises one or more viable cells, i.e. cells that display an intact cytoplasmic membrane functional synthesis of protein and other cells components such as nucleic acids, polysaccharides, etc., and energy production necessary to maintain cellular metabolism, and eventually, growth and multiplication.

As such, and without being bound by theory, the strain of the invention may further be qualified, to some extent, as a probiotic, in that it is capable of conferring a health benefit to a host in need, by notably controlling vibriosis in aquatic animals in need thereof, and accordingly e.g. reduce the mortality rate and/or increase the survival rate of the host.

"*Vibriosis*" refers to a disease caused by a group of virulent bacteria belonging to the *Vibrio* genus, also referred herein as virulent vibrios. Bacteria of the *Vibrio* genus are gram negative bacteria that can be found not only in marine environment and estuarine ecosystems, but also in aquaculture farms, either free-living, as part of a biofilm, or in association with a host. Vibriosis thus typically affects aquatic animals.

By "*aquatic animals*", it is meant herein animals that can survive and/or live in an aquatic environment such as salt and/or fresh water, and can be bred in aquaculture. Aquatic animals include, but are not limited to, marine animals such as molluscs, fishes, and crustaceans. For example, molluscs can include bivalve molluscs such as clams, oysters, scallops, mussels, or gastropods molluscs such as abalones. For example, fishes can include, without limitation, osteichthyes (e.g. trout, salmon, perch, bass, tuna, swordfish, marlin, grouper, sturgeon, wahoo, snapper, eel, walleye, catfish, tilapia, etc.) and chondrichthyes (e.g. sharks, rays, skates, etc.); crustaceans can include e.g. crabs, lobsters, crayfish, shrimp, krill, prawns, etc.

Preferred aquatic animals according to the invention are molluscs, in particular clams. For example, clams can be selected from the group of consisting of the following species: *Venerupis sp., Ruditapes sp., Venus sp., Circomphalus sp., Mercenaria sp., Callista sp., Amiantis sp., Meretrix sp., Mercentrica sp.,* and *Anadara sp.* More preferably, clams according to the invention are *Venerupis philippinarum*, also known as *Ruditapes philippinarum* or Manila clams.

Vibriosis can affect aquatic animals at any stage of their life cycle, such as the larval, post-larval, juvenile, or adult stage. These stages may be referred under different names depending on the species of aquatic animals (Mercer et al. Biological report 1989, 82(11.109), TR-EL-82-4; De Raedemaecker et al., Journal of the Marine Biological Association of the United Kingdom 2011; 91(183):21; Galappaththi, *University Of Manitoba* (165):8). Larval, post-larval and juvenile aquatic animals are particularly sensitive to vibriosis, especially when bred in aquaculture.

In a preferred embodiment, the aquatic animals according to the invention are, or intended to be, in aquaculture.

The term *"aquaculture*", "*aquaculturing*", "*aquafarm*", or "*aquafarming*" refers herein to the set of technical activities related to the culture, breeding, raising, production, propagation and/or harvesting of aquatic plants and/or animal species. Aquaculture may be used for production of food or raw materials for industrial and/or pharmaceutical use, or for the production of living organisms for repopulation purpose. Aquaculture methods may rely on seawater, brackish water, or freshwater, and may be implemented for monoculture (breeding of only one aquatic species) or integrated culture (culture in which the byproducts from one species are recycled to become input for another species). Aquaculture facilities can include, without limitation, hatching facilities, ponds, tanks, aquariums, pools, lakes, estuaries, oceans, marshes, lagoons, stretches of natural game fish waters and marine fish farms.

Vibriosis affecting aquatic animals can be responsible for their mass mortality, which in turn can result in severe economic loss when bred in aquaculture. In molluscs, typical clinical signs of vibriosis, notably in larvae, are reduction of larval motility, abnormal circular pattern of swimming and/or tendency to the quiescence by the inability to swim; at the peak of infection, dead and moribund larvae can exhibit bacteria swarming on the margins and inside, disruption and/or extension of the velum, detachment of portions of the velum with ciliary action after all other soft tissues are destroyed. In fishes, common symptoms of vibriosis can include intestinal necrosis, anemia, ascetic fluid, petechial hemorrhages in the muscle wall, and/or liquid in the air bladder, etc. In crustaceans, typical symptoms of vibriosis are mass mortalities more particularly observed at larval and post-larval stages.

Generally speaking, the term *"control* or *"controlling"* with regard to a disease means obtaining a beneficial biological effect depending on the degree of severity of the symptom or disorder of interest, or risk thereof, i.e. herein, depending on the degree of severity of vibriosis, or risk of developing such symptom or disorder. This beneficial effect may be prophylactic in terms of a partial or complete prevention of the symptom or disorder. In this context, "*control*" or "*controlling*" means "*preventing* "*prevention*", "*prophylaxis*" or *"prophylactic",* i.e. it characterizes the capacity to avoid, or minimize the onset or development of a symptom or disorder before its onset (for example, before the onset of vibriosis). Alternatively, the beneficial effect may be therapeutic in terms of a partial or complete cure of the disease. In this context, *"control"* or *"controlling"* means "*treating*"*, "therapy", "therapeutic",* i.e. it characterizes the capacity to inhibit the symptom or disorder (i.e. arresting the development thereof), and/or to relieve said symptom or disorder (i.e. regression leading to an improvement). In the context of the invention, a prophylactic effect is generally said to be achieved when e.g. clinical signs as described above are avoided when implementing the invention, while a therapeutic effect is typically said to be achieved when e.g. clinical signs as described above are reduced when implementing the invention. Accordingly, the aquatic animals in need of disease control according to the invention may be suffering from vibriosis, or be at risk thereof.

To this day, the *Vibrio* genus is known to include over eighty defined species, among which at least fifteen are estimated to be pathogenic (i.e. virulent) towards aquatic animals. These include *Vibrio tapetis*, *Vibrio harveyi, Vibrio alginolyticus, Vibrio aestuarianus*, *Vibrio coralliilyticus*, *Vibrio neptunius, Vibrio ostreicida*, *Vibrio bivalvicida, Vibrio europaeus*, *Vibrio tubiashii, Vibrio pectenecida*, *Vibrio crassostreae, Vibrio splendidus, Vibrio tasmaniensis, Vibrio parahaemolyticus, Vibrio anguillarum*, *Vibrio vulnificus, Vibrio ordalii*, *Vibrio salmonicida*, *Vibrio viscosus, Vibrio damsela*, *Vibrio fischeri, Vibrio vulnificus, Vibrio pelagius,* to name a few (Dubert et al. Frontiers in Microbiology, 2017, 8(462): 1-16; Karunasagar et al., Aquaculture 1994, 128 : 203-209; Mohamad et al., Aquaculture 2019, 512 : 1-17). These different *Vibrio* species can be easily identified using well-known methods in the art such as molecular techniques in bacterial taxonomy, including, *inter alia*, sequencing of 16S rRNA and/or other housekeeping genes, ribotyping, PCR-based techniques such as arbitrarily primed PCR (AP-PCR), species-specific primed-PCR (SSP-PCR), repetitive extragenic palindrome (rep)-PCR (rep-PCR), amplified fragment length polymorphism (AFLP), fluorescence *in situ* hybridization (FISH), randomly amplified polymorphic DNA (RAPD), and restriction fragment length polymorphism (RFLP) (Chatterjee and Haldar, 2012, J Marine Sci Res Dev, S:1).

Because of the prevalence *Vibrio* bacteria in a wide range of aquatic animals, it is thought that this type of bacteria is capable of crossing species barriers. This is notably the case for *V. tapetis* as reported by Paillard in 2016 (Oysters and Clams: Cultivation, Habitat Threats and Ecological Impact, Nova Science Publishers, 2016, 978-1-63485-074-2, hal-01426301). It shall nevertheless be understood that not all bacteria of the *Vibrio* species are pathogenic, as herein demonstrated by the Inventors with the *V. tapetis* strain deposited on the 6^{th} January 2021 at the CNCM under the accession number CNCM I-5635; said strain is indeed avirulent or substantially avirulent towards aquatic animals, such as towards clams, fishes or crustaceans.

The term "*avirulent*", "*non-virulent*" or "*non-pathogenic*" as used herein, does not mean that a bacterium of that genus or species cannot ever function as a pathogen, but that the particular bacterium is avirulent with respect to a particular animal. The bacterium may belong to a genus or even a species that is normally pathogenic but must belong to a strain that is avirulent. An "*avirulent strain*" is thus incapable of inducing the full set of symptoms of the disease that is normally associated with its pathogenic counterpart. In contrast, "*pathogenic*" or "*virulent*" as used herein, means that the capable of causing disease or impairing normal physiological functioning.

"*Vibrio tapetis*"*,* also referred as "*V. tapetis*" is a gram-negative, marine rod-shaped bacterium, which can be easily identified by conventional methods in the art as mentioned above. Paillard et al. developed in 2006 universal tools for the molecular identification of all *V. tapetis* strains (namely a specific 16S rDNA probe and species-specific primers) (Aquaculture, 2006, 253(1-4): 25-38). This is only in 2018 that the structural feature differentiating virulent and avirulent *V. tapetis* was discovered: a Type IV Secretion System, referred as T4SS that comprise a *vir* operon (*virB2, virB3, virB4, virB6, virb9, virB10, virB11*). A particular component of the Type IV Secretion System, referred as *virB4,* was notably shown to be present in the genome of virulent strains, but absent in non-pathogenic strains (Dias et al., 2018, Frontiers in Microbiology, 9:227: 1-14). Non limitative examples of *V*. *tapetis* strains comprising a T4SS are the *V. tapetis* strains CECT4600, IS-1, IS-5, IS-7, IS-8, IS-9, RP2.2, RP8.17, RP9.7, RP11.2, UK6, RD0705 and P16B. From a functional standpoint, virulent *V*. *tapetis* strains can be identified by *in vivo* or *in vitro* virulence assays, such as the assays developed for clams by Paillard and Maes (Dis. Aquat. Organ., 1994, 19:137-146), Oubella et al. (Journal of invertebrate pathology, 1994, 64:33-38) and Choquet et al. in 2003 (Dis. Aquat. Org., 57:109-116). For illustrative purpose, the *in vitro* virulence assays are typically based on the observations that virulent *V*. *tapetis* strains can affect clam hemocytes by causing cell-rounding, loss of cytoplasmic extension and, as a consequence, loss of adherence capacity.

Accordingly, in a preferred embodiment of the present invention, the vibriosis is mediated by a virulent *Vibrio tapetis* strain comprising a Type IV Secretion System (T4SS), especially when the aquatic animals in need of disease control are molluscs, such as clams.

In a more preferred embodiment, the vibriosis is mediated by the virulent *Vibrio tapetis* CECT4600 strain, especially when the aquatic animals in need of disease control are molluscs such as clams, in particular *Venerupis sp.* clams such as *Venerupis philippinarum.* The *Vibrio tapetis* CECT4600 strain, also referred as ATCC 700075, DSM 21475, or LMG 19706 in culture collections, and formerly named *Vibrio* VP1, is an agent responsible for a vibriosis referred as the Brown Ring Disease (BRD). The genome of the *Vibrio tapetis* CECT4600 strain has been fully characterized for the first time in 2018 (Dias et al., 2018, Frontiers in Microbiology, 9:227: 1-14).

Thus, in a preferred embodiment, the vibriosis to be controlled is Brown Ring Disease (BRD), especially when the aquatic animals in need of disease control are molluscs such as clams, in particular *Venerupis sp.* clams such as *Venerupis philippinarum.*

"*Brown Ring Disease*" is a vibriosis mediated by the pathogen *Vibrio tapetis,* in particular by the CECT4600 strain, and which mainly affects cultured and wild populations of clams. BRD typically induces the formation of a brown deposit on inner shell of the clams, and leads to depressed defense-associated activities in the infected clams.

For the purpose of the invention, it should be further understood that the isolated *Vibrio tapetis* strain deposited on the 6^{th} January 2021 at the CNCM under the accession number CNCM 1-5635 is provided in an amount sufficient to control vibriosis in aquatic animals in need thereof.

Accordingly, in a preferred embodiment, the isolated *Vibrio tapetis* strain deposited on the 6^{th} January 2021 at the CNCM under the accession number CNCM 1-5635 is used at an effective amount.

"*By effective amount*", it is meant that the amount of said bacterial strain is sufficient for its intended use. Such effective amount can be determined based for example on the cellular density of the respective isolated *Vibrio tapetis* strain deposited on the 6^{th} January 2021 at the CNCM under the accession number CNCM 1-5635 and the pathogenic *Vibrio tapetis* strain.

By "*cellular density*", *"density",* or "*quantitative distribution*"*,* it is meant herein the cells number per volume (e.g. cells/ml, cells/L, CFU/ml, etc), within the environment comprising, or intended to comprise, the aquatic animals (i.e. aquatic environment). Said density can be easily determined by the skilled person in the art using conventional methods in the art, such as by optic density measurement at a wavelength of about 492 nm.

In a preferred embodiment, the isolated *V*. *tapetis* strain deposited on the 6^{th} January 2021 at the CNCM under the accession number CNCM 1-5635 is preferably used at the same or higher, preferably higher, cellular density than the one of the virulent *Vibrio tapetis* strain that induces the vibriosis in the aquatic animals.

For example, the ratio R between the cellular density of the virulent *Vibrio tapetis* strain that induces the vibriosis in the aquatic animals and the cellular density of the isolated *Vibrio tapetis* strain deposited on the 6^{th} January 2021 at the CNCM under the accession number CNCM 1-5635 is of about 1:1, 1:2, 1:3, 1:4, 1:5, 1:6, 1:7, 1:8, 1:9, or 1:10, preferably of about 1:2, 1:3, 1:4, 1:5, 1:6, 1:7, 1:8, 1:9, or 1:10, more preferably of about 1:3, 1:4 or 1:5, even more preferably of about 1:3 or 1:5.

In another aspect, the invention is directed to an isolated *Vibrio tapetis* strain deposited on the 6^{th} January 2021 at the CNCM under the accession number CNCM 1-5635, or a composition thereof, for use in promoting the health of aquatic animals in need thereof.

In particular, the present invention is directed to the use of an isolated *Vibrio tapetis* strain deposited on the 6^{th} January 2021 at the CNCM under the accession number CNCM 1-5635, or of a composition thereof, for the preparation of an active agent, such as a medicament, for promoting the health of aquatic animals in need thereof.

It is also described herein a method for promoting the health of aquatic animals in need thereof, said method comprising the step of adding an effective amount of an isolated *Vibrio tapetis* strain deposited on the 6^{th} January 2021 at the CNCM under the accession number CNCM 1-5635, or of a composition thereof, to an environment comprising or intended to comprise aquatic animals (i.e. an aquatic environment).

Preferred embodiments as described above apply herein *mutatis mutandis.*

By "*promoting the health*" or "*enhancing the health*" of aquatic animals, it is meant herein to improve the general physical condition of such animals. Indicia of enhanced health include, without limitation, increase in growth or proliferation, increase in productivity, increase in disease resistance, increase in survival rate, reduction in risk of mortality, and/or reduction of morbidity, to name a few.

In another aspect, the invention relates to the use of an isolated *Vibrio tapetis* strain deposited on the 6^{th} January 2021 at the CNCM under the accession number CNCM 1-5635, or of a composition thereof, for controlling virulent *Vibrio tapetis* strains in an aquatic environment such as in aquaculture.

In particular, the invention relates to a method for controlling virulent *Vibrio tapetis* strains in an aquatic environment such as in aquaculture, said method comprising the step of adding, in the environment, an isolated *Vibrio tapetis* strain deposited on the 6^{th} January 2021 at the CNCM under the accession number CNCM 1-5635, or a composition thereof.

Preferred embodiments as described above apply herein *mutatis mutandis.*

The term *"control* or "*controlling*" with regard to a pathogen means obtaining a beneficial biological effect depending on the degree of presence of the pathogen in an environment, or risk thereof, i.e. herein, depending on the degree of presence of virulent *Vibrio tapetis* strain in the aquatic environment, or risk thereof. This beneficial effect may allow a partial or complete prevention of the presence of the pathogen in the environment. In this context, "*control*" or "*controlling*" means "*preventing*" or "*prevention*", i.e. it characterizes the capacity to avoid, or minimize the presence of the pathogen in the environment. Alternatively, the beneficial effect may allow a partial or complete elimination of the pathogen in the environment, i.e. when the pathogen is already present in said environment. In this context, "*control*" or "*controlling*" means "*reducing*", "*reduction*"*,* "*inhibiting*" or "*inhibition*"*,* i.e. it characterizes the capacity to inhibit or reduce the proliferation or growth of the pathogen in the environment. In other words, *"control* or *"controlling"* a pathogen means control or controlling the presence, proliferation or growth of said pathogen in the environment.

In another aspect, the invention relates to the use of an isolated *Vibrio tapetis* strain deposited on the 6^{th} January 2021 at the CNCM under the accession number CNCM 1-5635, or of a composition thereof, in the culturing of aquatic animals, such as in aquaculture.

In particular, the present invention is directed to a method for culturing aquatic animals, comprising the step of adding, in an aquatic environment such as in an aquaculture, an isolated *Vibrio tapetis* strain deposited on the 6^{th} January 2021 at the CNCM under the accession number CNCM 1-5635, or a composition thereof.

Preferred embodiments as described above apply herein *mutatis mutandis.*

In another aspect, the invention pertains to an isolated *Vibrio tapetis* strain deposited on the 6^{th} January 2021 at the CNCM under the accession number CNCM 1-5635. As mentioned above, said strain is particularly useful for promoting the health and welfare of aquatic organisms, for controlling vibriosis in aquatic animals in need thereof, for controlling virulent *Vibrio tapetis* strains in aquaculture, and/or for culturing aquatic animals.

In a preferred embodiment, the isolated *Vibrio tapetis* strain deposited on the 6^{th} January 2021 at the CNCM under the accession number CNCM 1-5635 is in a live form.

It is another aspect of the present invention to provide a composition comprising at least an isolated *Vibrio tapetis* strain deposited on the 6^{th} January 2021 at the CNCM under the accession number CNCM 1-5635 as an active agent.

The composition of the invention may be in various forms, such as in the form of a liquid (e.g. aqueous solution, etc.), semi-solid (e.g. moist pellets, etc.), solid (e.g. pills, capsules, granules, tablets, etc.) or lyophilized formulation (e.g. wettable powder, dry powder, etc.). To do so, the composition may preferably further comprise a suitable carrier or excipient, such as a pharmaceutically or veterinary or food acceptable carrier or excipient.

The carrier or excipient present in the composition is herein intended to allow the bacterial active agent to remain efficacious (e.g., capable of improving aquatic animal well-being, general condition, health or survival rate, more particularly capable of controlling vibriosis in aquatic animals and/or of controlling virulent *Vibrio tapetis* strains in an aquatic environment) and preferably viable once formulated in the composition. It shall also be understood that said carrier or excipient is preferably harmless to aquatic animals.

Suitable excipients or carriers according to the invention include, without limitation, diluents, buffers, preservatives, surface active agents, thickeners, and the like.

For example, for liquid formulations, saline, sterile water such as filtered and sterilized seawater that has been filtered and sterilized, sugar water, Ringer's solution, buffered physiological saline, albumin infusion solution, dextrose solution, maltodextrin solution, glycerol, ethanol, or mixtures thereof may be used as a carrier or excipient. If a liquid carrier or excipient is used, the composition may further include growth media to culture the bacterial active agent. Non-limiting examples of suitable growth media for the deposited bacterial strain include Luria Broth with Salt (LBS) medium, Zobell medium, Marine broth and Marine agar medium, or any media known to those skilled in the art to be compatible with, and/or provide growth nutrients to said strain.

As another example, for solid formulations, calcium carbonate, sodium bicarbonate, sodium chloride, peat, wheat, wheat chaff, ground wheat straw, bran, vermiculite, cellulose, starch, soil (pasteurized or unpasteurized), gypsum, talc, clays (e.g., kaolin, bentonite, montmorillonite), and silica gels may be used as a carrier or excipient.

In a preferred embodiment, the isolated *Vibrio tapetis* strain deposited on the 6^{th} January 2021 at the CNCM under the accession number CNCM 1-5635 is present in the composition in a live form.

It should be further understood that the composition of the invention may comprise an effective amount of the isolated *Vibrio tapetis* strain deposited on the 6^{th} January 2021 at the CNCM under the accession number CNCM 1-5635.

In a preferred embodiment, the composition of the invention is an additive intended for culturing aquatic animals. For example, the composition of the invention may be added to or administered with feed for aquatic animals, and as such may be referred as a feed additive.

In a preferred embodiment, the composition of the invention is a probiotic composition.

The present invention will be better understood in the light of the following detailed experiments. Nevertheless, the skilled artisan will appreciate that the present examples are not limitative and that various modifications, substitutions, omissions, and changes may be made without departing from the scope of the invention.

### EXAMPLES

### MATERIALS AND METHODS

### Bacterial strains, construction and culture conditions

*V. tapetis* strains used in this study are described in the following Table 1.

**Table 1. V. tapetis bacterial strains**

| **Strains** | **Description** |
|---|---|
| CECT4600 | Virulent *V. tapetis* strain (wild type) isolated form *R. philippinarum* ( Borrego JJ et al., 1996, International Journal of Systematic and Evolutionary Microbiology (1996) 46:480-484.) |
| GDE | Avirulent *V. tapetis* strain for the Manila clam (Dias et al., 2018, Frontiers in Microbiology, 9:227: 1-14) |
| CECT4600 - TrimR | Virulent *CECT4600 V. tapetis strain resistant to Trimethoprim* |
| GDE-KmR | Avirulent *V. tapetis* strain for the Manila clam resistant to kanamycin |

*E. coli* strains were routinely grown at 37°C in LB medium. The CECT4600 and GDE *V*. *tapetis* strains were grown in LB medium containing 20 g/l (final concentration) of NaCl (LBS) or in Zobell medium at 18°C (Zobell C. J Mar Res (1994) 4:41-75). When necessary, the following components were added: Kanamycin (Km, 100 µg/ml for *E. coli* and 250 µg/ml for *V*. *tapetis),* Trimethoprim (Trim, 10 µg/ml), or agar (15 g/l).

### In vitro competition of bacterial growth

The *V*. *tapetis* strains were plated on LBS-Agar media supplemented with the appropriate antibiotic. Overnight pre-cultures were run at 18°C in LBS. Optical densities were measured to determine the concentrations of each pre-culture at a wavelength of 492 nm, and cell densities were adjusted at 10⁵ CFU/ml. Initial cultures have been made using different proportions (ratios) of each pre-culture according to the pre-mix distribution detailed in the following Table 2.

**Table 2. Proportions of each V.tapetis strain used (CECT4600+pFD092 and GDE-KmR) for the in vitro competition assay**

| **Conditions** | **Volume of LBS** | **Volume of CECT4600 suspension at 10⁹ CFU/ml** | **Volume of GDE suspension 10⁹ CFU/ml** | **Total volume** |
|---|---|---|---|---|
| Control LBS | 10 ml | - | - | 10 ml |
| CECT4600 (1:0) | 9 ml | 1 ml | - | 10 ml |
| GDE (0:1) | 9 ml | - | 1 ml | 10 ml |
| GDE (0:3) | 7 ml | - | 3 ml | 10 ml |
| GDE (0:5) | 5 ml | - | 5 ml | 10 ml |
| CECT4600 : GDE (1:1) | 8 ml | 1 ml | 1 ml | 10 ml |
| CECT4600 : GDE (1:3) | 6 ml | 1 ml | 3 ml | 10 ml |
| CECT4600 : GDE (1:5) | 4 ml | 1 ml | 5 ml | 10 ml |

Growth parameters of the *V*. *tapetis* strains CECT4600-TrimR and GDE-KmR were determined by using a microplate reader (BioscreenC) at 18°C with agitation, in 100-well microplates, after 24 h of growth, in triplicates.

To determine the concentration of each strain at the beginning of the culture (T0), each initial culture was serially diluted (until 10⁻⁸-fold) and plated on either LBS+Trim or LBS+Km medium. At the end of the microplate cultures, the concentrations of each strain were determined in the same way. Plates were incubated at 18°C and colonies were enumerated for each dilution whenever possible.

### In vitro virulence assay on Manila clams

The *in vitro* virulence assay was performed, following a standardized protocol developed by Choquet et al. (Diseases of aquatic organisms (2003) 57:109-116). This allows to test the ability of *V*. *tapetis* strains to induce a loss of adhesion properties on hemocytes of the Manila clam, this loss of adhesion being an indicator of cytotoxic activity. The results are typically expressed as a non-adherent cell ratio, i.e. the number of non-adherent hemocytes incubated with a bacterium divided by the number of hemocytes incubated with FSSW. A non-adherent cell ratio statistically above 1 represents a cytotoxic effect of the tested bacterial strain. Briefly, Manila clams from the SATMAR shellfish aquaculture site in Marennes (Charente-Maritime, France) were acclimatized in oxygenated seawater at 14°C. Hemolymph was harvested directly from the adductor muscle of each individual, and the quality of hemocytes was checked by microscopical observation (presence of pseudopods, number of rounded hemocytes). Hemolymph samples of good quality were pooled together and the hemocytes enumerated by using a Malassez cell. Hemolymph was exposed either to a bacterial suspension, or only to sterile-filtered seawater (FSSW) as a negative control. Bacterial suspensions were prepared as described in the following Table 3 in order to expose hemocytes to different proportions of mixed virulent (CECT4600 strain) /avirulent (GDE strain) strains while maintaining a bacteria: hemocyte ratio of 25:1 per exposure of hemolymph to bacterial suspension.

**Table 3. Proportions of each V.tapetis strain used for the in vitro virulence assay on Manila clams**

| **Conditions** | **Volume of FSSW** | **Volume of CECT4600 suspension** | **Volume of GDE suspension** | **Ratio bacteria: hemocyte** |
|---|---|---|---|---|
| Control FSSW | 110 µL | - | - | FSSW: 0 :1 |
| CECT4600:GDE (1:0) | 100 µL | 10 µL | | CECT4600 : 25:1 |
| CECT4600: GDE (0:1) | 100 µL | - | 10 µL | GDE: 25:1 |
| CECT4600 : GDE (1:1) | 90 µL | 10 µL | 10 µL | CECT4600 : 25:1 GDE: 25:1 |
| CECT4600 : GDE (1:3) | 70 µL | 10 µL | 30 µL | CECT4600 : 25:1 GDE: 75:1 |
| CECT4600 : GDE (1:10) | - | 10 µL | 100 µL | CECT4600 : 25:1 GDE : 250:1 |
| CECT4600:GDE (0:3) | 80 µL | - | 30 µL | GDE: 75:1 |

To do so, 100 µl of hemolymph were added in 24-well plates, followed by 110 µl of bacterial suspension as described in Table 3, in triplicates. The concentration of the CECT4600 strain was adjusted to obtain a 25:1 ratio of bacteria: hemocyte for each condition tested. For the FSSW negative controls, the bacterial suspension was replaced by 110 µl of FSSW. After a 3h-incubation at 18°C, each well was then fixed with formol (3% final concentration). The ratio of non-adherent hemocytes was determined using flow cytometry by adding 2.5 µL of SYBR Green 10⁻¹ to 250 µL of tested sample. Results were expressed as the ratio of non-adherent hemocytes in exposed versus control samples.

### In vivo virulence assay on Manila clams

For the *in vivo* experiments, Manila clams were infected by inoculating bacteria into the pallial cavity in order to induce Brown Rind Disease (BRD) as described by Oubella et al. (Journal of invertebrate pathology (1994) 64:33-38). Briefly, the day prior to the injection, clams were removed from water to allow them to open enough for injection the next day. Around 150 clams were injected with 100 µl of a suspension (10⁷ CFU/animal) of the CECT4600 *V. tapetis* strain, the GDE *V. tapetis* strain, a combination of both strains, or with 100 µl FSSW as a negative control, as described in the following Table 4.

**Table 4. Proportions of each V.tapetis strain used for the in vivo virulence assay on Manila clams**

| **Conditions** | **Volume of FSSW** | **Volume of CECT4600 suspension at 10⁹ CFU/ml** | **Volume of GDE suspension 10⁹ CFU/ml** | **Total volume** |
|---|---|---|---|---|
| Control FSSW | 10 ml | - | - | 10 ml |
| CECT4600:GDE (1:0) | 9 ml | 1 ml | - | 10 ml |
| CECT4600: GDE (0:1) | 9 ml | - | 1ml | 10 ml |
| CECT4600: GDE (0:3) | 7 ml | - | 3 ml | 10 ml |
| CECT4600: GDE (0:5) | 5 ml | - | 5 ml | 10 ml |
| CECT4600 : GDE (1:1) | 8 ml | 1 ml | 1ml | 10 ml |
| CECT4600 : GDE (1:3) | 6 ml | 1 ml | 3 ml | 10 ml |
| CECT4600 : GDE (1:5) | 4 ml | 1 ml | 5 ml | 10 ml |

To this end, all injected clams were left for 6h out of water to promote the colonization of the periostracal lamina by *V*. *tapetis.* Animals were then placed into tanks (50 animals per replicate, 3 replicates for each condition) equipped with a bubbler, at 14°C. Animals were not fed during the experiment and water was not changed post injection (p.i.). The replicates were injected in 3 independent days. After 4 weeks p.i., animals were sacrificed and shells were kept and analyzed according to the method developed by Paillard et al. (Diseases of aquatic organisms (1994) 19:137-146) to determine BRD prevalence in each condition. To determine whether the BRD prevalence was statistically significant between these conditions, a Chi-2 test with 2 degree of freedom was performed.

Three independent experiments were performed according to this protocol to determine the prevalence of Brown Ring Disease following co-exposure of the clams to the CECT4600 and GDE strains at the 1:1; 1:3 and 1:5 ratios. The BRD extent index, which determines the progress of the infection (only in clams presenting BRD signs), was further determined as described by Paillard et al. (Diseases of aquatic organisms (1994) 19:137-146).

### RESULTS

### Aim of the study

The aim of the present study was to investigate the effect, in the context of BRD, of the GDE *V*. *tapetis* strain on vibriosis affecting aquatic animals. To this end, the study was conducted in a model of the Brown Ring Disease, caused by the pathogenic bacterium *Vibrio tapetis* CECT4600 strain, in the Manila clam *Ruditapes philippinarum.*

### In vitro competition of bacterial growth

Results of *in vitro* competition in bacterial growth assay are presented on Figure 1. Concentration of the GDE strain was higher than the one of the CECT4600 strain, both at T0 and TF (i.e. 24h), whether the GDE strain was in co-culture or not. Nevertheless, co-culturing the GDE strain with the CECT4600 strain reduced the growth of CECT4600 by at least 5-fold. This result indicates that the GDE strain negatively impacts the growth of the pathogenic CECT4600 strain.

### In vitro virulence assay on Manila clams

Results of the *in vitro* virulence assay are illustrated on Figure 2.

Exposure of Manila clam hemocytes to the sole pathogenic bacterium *V*. *tapetis* CECT4600 at a hemocyte:bacteria ratio of 1:25 revealed, as expected, a significative increase in the non-adherent hemocytes ratio (*pvalue:* 0.00029). By contrast, exposure of the clam hemocytes to the sole GDE *V*. *tapetis* strain at hemocyte:bacteria ratio of 1:25 did not modify the non-adherent hemocyte ratio, or at least not significantly; similar results were observed when increasing the hemocyte: GDE bacteria ratio to 1:75.

It was then assessed whether the GDE strain could counteract the cytotoxic effect of the CECT4600 strain by co-exposing clam hemocytes to both strains. This co-exposure reduced the ratio of non-adherent hemocytes compared to Manila clam hemocytes exposed solely to the CECT4600 strain. This reduction increased when augmenting the proportion of GDE strain (ratios 1:1 and 1:3 of the CECT4600: GDE strains were tested).

### In vivo virulence assays on Manila clams

In order to assess the ability of the *V*. *tapetis* GDE strain to reduce BRD prevalence induced by the *V*. *tapetis* CECT4600 strain, several independent experiments were performed by co-exposing Manila clams to both *V*. *tapetis* at different ratios (1:1, 1:3 and 1:5).

Proportion of BRD-positive Manila clams injected with either FSSW (negative control) or the sole *V*. *tapetis* GDE strain was low, with no significant difference. After exposure to the pathogenic CECT4600 *V*. *tapetis* strain, the percentage of BRD observed was comprised between 63% and 88%; BRD prevalence was significantly reduced when the GDE strain was injected concomitantly with the virulent CECT4600 strain, for all the ratios tested, by at least about 20% (ratio 1:1), and up to 64% (ratio 1:3). It should nevertheless be noted that BRD prevalence is very dependent on clams' conditions, impacted by seasons and also by the pool of clams used to perform the experiment. For these reasons, in order to compare the efficiency of the different ratios, only the ones tested in the same experiment should be compared with each other. In other words, to compare the efficiency of ratios 1:3 and 1:5, one should only look at experiment 3. In this last experiment, Figure 3A (graph at the bottom) shows that the GDE strain reduces BRD prevalence by 50%, at both ratios, i.e. no significant difference was detected between the ratios 1:3 and 1:5. The extent index of infected clams in experiment 3 was thus calculated in order to determine the progress of the infection. The results, reported on Figure 3B, show that, despite a similar reduction in BRD prevalence at co-exposure ratios of 1:3 and 1:5 in experiment 3, the infection was far less advanced in BRD-positive clams with the 1:5 ratio than with the 1:3 ratio. This means that increasing the proportion of the GDE strain has a beneficial effect for treating vibriosis induced by the pathogenic CECT4600 *V.tapetis* strain, as it can slow down disease progression.

### CONCLUSION

The present study is the first of its kind to investigate the beneficial effect against BRD of the avirulent GDE strain. Overall, the results of this study demonstrate the capacity of the *Vibrio tapetis* GDE strain to significantly reduce the cytotoxic effects induced by the pathogenic *Vibrio tapetis* strain responsible for BRD, and accordingly reduce BRD prevalence as well as the BRD extent index. The GDE strain can accordingly be used as a new agent for controlling vibriosis in aquatic animals, such as BRD in Manila clams.

## Claims

1. An isolated *Vibrio tapetis* strain deposited on the 6^{th} January 2021 at the CNCM under the accession number CNCM 1-5635 for use in controlling vibriosis in aquatic animals in need thereof.

2. The isolated *Vibrio tapetis* strain for use according to claim 1, wherein the aquatic animals are molluscs, fishes or crustaceans, preferably molluscs.

3. The isolated *Vibrio tapetis* strain for use according to claim 2, wherein the molluscs are clams, preferably *Venerupis sp.* clams, more preferably *Venerupis philippinarum.*

4. The isolated *Vibrio tapetis* strain for use according to any one of the preceding claims, wherein the aquatic animals are, or intended to be, in aquaculture.

5. The isolated *Vibrio tapetis* strain for use according to any one of the preceding claims, wherein the vibriosis is mediated by a virulent *Vibrio tapetis* strain, preferably by the virulent *Vibrio tapetis* CECT4600 strain.

6. The isolated *Vibrio tapetis* strain for use according to claim 5, wherein the vibriosis is Brown Ring Disease (BRD).

7. The isolated *Vibrio tapetis* strain for use according to any one of the preceding claims, wherein the isolated *Vibrio tapetis* strain deposited on the 6^{th} January 2021 at the CNCM under the accession number CNCM 1-5635 is in a live form.

8. The isolated *Vibrio tapetis* strain for use according to any one of the preceding claims, wherein the isolated *Vibrio tapetis* strain deposited on the 6^{th} January 2021 at the CNCM under the accession number CNCM 1-5635 is used at the same or higher cellular density than the one of the virulent *Vibrio tapetis* strain that induces the vibriosis in the aquatic animals.

9. An isolated *Vibrio tapetis* strain deposited on the 6^{th} January 2021 at the CNCM under the accession number CNCM 1-5635, for use in promoting the health of aquatic animals in need thereof.

10. Use of an isolated *Vibrio tapetis* strain deposited on the 6^{th} January 2021 at the CNCM under the accession number CNCM 1-5635, for controlling virulent *Vibrio tapetis* strains, preferably the virulent *Vibrio tapetis* CECT4600 strain, in an aquatic environment, such as in aquaculture.

11. Use of an isolated *Vibrio tapetis* strain deposited on the 6^{th} January 2021 at the CNCM under the accession number CNCM 1-5635, in the culturing of aquatic animals, such as in aquaculture.

12. An isolated *Vibrio tapetis* strain deposited on the 6^{th} January 2021 at the CNCM under the accession number CNCM 1-5635.

13. A composition comprising at least an isolated *Vibrio tapetis* strain deposited on the 6^{th} January 2021 at the CNCM under the accession number CNCM 1-5635 as an active agent.

14. The composition of claim 13, wherein said composition is an additive intended for culturing aquatic animals, such as a feed additive.

15. The composition of claim 13 or 14, wherein said composition is a probiotic composition.
